# EUROPÄISCHE PATENTSCHRIFT

(11) **EP 2 377 844 B1**
(45) Veröffentlichungstag und Bekanntmachung des Hinweises auf die Patenterteilung: **18.10.2017**
(21) Anmeldenummer: 10177776.1
(22) Anmeldetag: 21.06.2005
(51) Int. Cl.: C07C 67/303, C07C 69/75, C08K 5/12, C09J 11/06

(54) **Hilfsmittel enthaltend Cyclohexanpolycarbonsäurederivate**
Cyclohexane polycarboxylic acid derivatives containing adjuvants
Moyen d'aide contenant des dérivés d'acide cyclohexane-polycarboxylique

(30) Priorität: 21.06.2004 DE 102004029732
(43) Veröffentlichungstag der Anmeldung: 19.10.2011
(62) Teilanmeldung aus: 05769030.7
(73) Patentinhaber: BASF SE, 67056 Ludwigshafen am Rhein (DE)
(72) Erfinder: Storzum, Uwe, 67551 Worms (DE); Breitscheidel, Boris, 67117 Limburgerhof (DE)

(56) Entgegenhaltungen:
- WO-A1-03/029339

## Beschreibung

Die vorliegende Erfindung betrifft Tapeten, Bodenbeläge aus Linoleum, Gummi oder Textil, Parkett oder Isolierglasfenster enthaltend einen Klebstoff, enthaltend mindestens einen 1,2-Cyclohexandicarbonsäureester ausgewählt aus der Gruppe bestehend aus 1,2-Diisobutylcyclohexandicarbonsäureester, 1,2-Di-(2-ethylhexyl)cyclohexandicarbonsäureester und Diisononylcyclohexandicarbonsäureester und die Verwendung eines Klebstoffes in Tapeten, Bodenbelägen oder Isolierglasfenstern.

Aus EP-A 0 541 788 ist bekannt, Ester von Milchsäure und 2-Butoxyethanol, Ester von Phthalsäure und 2-(2-Butoxy)ethanol, Methylphenylcarbinol, Ethylenglykol, Diethylenglykol, Diacetonalkohol, Propylenglykol, Mono- und Diether von Ethylen- oder Propylenglykol oder Mischungen davon als Fließ- oder Filmbildungshilfen in Beschichtungsmittelzusammensetzungen einzusetzen. Diese Fließ- oder Filmbildungshilfen verbessern das Fließen und minimieren gleichzeitig die Schaumbildung. Einige dieser Fließhilfen sind jedoch toxikologisch bedenklich, so dass deren Ersatz durch toxikologisch unbedenkliche Verbindungen wünschenswert ist. Des Weiteren können Beschichtungsmittelzusammensetzungen zusätzlich innere Koaleszenzmittel enthalten, zum Beispiel Butyloctylphthalat. Ein Ersatz von Phthalaten als Koaleszenzmittel, die toxikologisch bedenklich sind, durch toxikologisch unbedenkliche Verbindungen ist ebenfalls wünschenswert.

EP-A 0 523 122 betrifft Schmierstoffzusammensetzungen, die synthetische Ester aus aliphatischen Polyolen und Monocarbonsäuren als Additive oder Ölphase enthalten. Es ist wünschenswert, die Palette an möglichen Additiven zu erweitern, um so die Eigenschaften von Schmierstoffzusammensetzungen optimal an deren Einsatzbedingungen anpassen zu können.

Entschäumer, Schaumverhüter und Benetzungsmittel enthalten häufig Phthalsäurediester. Diese sind jedoch aus umwelttechnischen Gründen ins Gerede gekommen, so dass ein Ersatz der Phthalsäurediester durch umwelttechnisch unbedenkliche Verbindungen wünschenswert ist.

Aufgabe der vorliegenden Anmeldung ist daher die Bereitstellung von toxikologisch unbedenklichen Verbindungen, die vielseitig als Hilfsmittel oder in Hilfsmitteln verwendbar sind und zum Ersatz von gegebenenfalls toxikologisch bedenklichen Additiven geeignet sind, ohne die Eigenschaften der Produkte, in denen die Hilfsmittel eingesetzt werden, negativ zu beeinflussen.

Die Aufgabe wird gelöst durch Tapeten, Bodenbeläge aus Linoleum, Gummi oder Textil, Parkett oder Isolierglasfenster enthaltend einen Klebstoff, enthaltend mindestens einen 1,2-Cyclohexandicarbonsäureester ausgewählt aus der Gruppe bestehend aus 1,2-Diisobutylcyclohexandicarbonsäureester, 1,2-Di-(2-ethylhexyl)cyclohexandicarbonsäureester und Diisononylcyclohexandicarbonsäureester.

Die Aufgabe wird ebenfalls durch die Verwendung eines Klebstoffes, wie erfindungsgemäß definiert, in Tapeten, Bodenbelägen aus Linoleum, Gummi oder Textil, Parkett, oder Isolierglasfenstern gelöst.

Bislang werden 1,2-Cyclohexandicarbonsäureester im Wesentlichen als Weichmacher für Kunststoffe, insbesondere PVC, eingesetzt (z.B. WO 00/78853), während ihr Einsatz in anderen technischen Bereichen bisher nahezu unbekannt ist. In den folgenden Dokumenten sind weitere Anwendungen von 1,2-Cyclohexandicarbonsäureestern in allgemeiner Form erwähnt:

DE-A 102 32 868 und DE-A 102 25 656 betreffen die Hydrierung von aromatischen Verbindungen, insbesondere die Herstellung von alicyclischen Carbonsäuren oder deren Estern durch Kernhydrierung der entsprechenden aromatischen Polycarbonsäuren oder deren Ester sowie geeignete Katalysatoren zur Hydrierung. Die mittels den in DE-A 102 32 868 und DE-A 102 25 656 offenbarten Hydrierverfahren hergestellten Polycarbonsäureester können neben einer Anwendung als Weichmacher als Schmierölkomponente, als Bestandteil von Kühlflüssigkeiten und Metallbearbeitungsflüssigkeiten verwendet werden. Des Weiteren können sie als Komponenten in Farben, Lacken, Tinten und Klebstoffen eingesetzt werden.

WO 03/029339 A1 beschreibt Weich-PVC, welches Cyclohexanpolycarbonsäureester als Weichmacher enthält.

In WO 03/029168 sind Gemische von alicyclischen Polycarbonsäureestern mit hohem cis-Anteil offenbart, die durch Kernhydrierung der entsprechenden aromatischen Polycarbonsäureester hergestellt werden. Neben einer Verwendung als Weichmacher ist in WO 03/029168 in allgemeiner Form die Verwendung der alicyclischen Polycarbonsäureester als Schmierölkomponente, als Bestandteil von Kühlflüssigkeiten und Metallbearbeitungsflüssigkeiten offenbart.

In WO 03/029181 sind Gemische von alicyclischen Polycarbonsäureestern mit einem Anteil der trans-Isomere von über 10 Mol-% offenbart, die durch Kernhydrierung der entsprechenden aromatischen Polycarbonsäureester hergestellt werden. Neben einer Verwendung als Weichmacher ist in WO 03/029181 in allgemeiner Form die Verwendung der alicyclischen Polycarbonsäureester als Schmierölkomponente, als Bestandteil von Kühlflüssigkeiten und Metallbearbeitungsflüssigkeiten offenbart.

JP-A 09 249 890 betrifft spezielle aliphatische cyclische Carbonsäureester als Gleitmittel in der Metallverarbeitung. Bei den aliphatischn cyclischen Carbonsäureestern kann es sich um Cyclohexan- oder Cyclohexendicarbonsäureester handeln.

Gemäß der vorliegenden Anmeldung wurde gefunden, dass spezielle 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester, bevorzugt 1,2-Diisononylcyclohexandicarbonsäureester, als oder in Hilfsmittel(n) für verschiedene bisher noch nicht im Stand der Technik genannte weitere Anwendungen geeignet sind.

Im Sinne der vorliegenden Erfindung geeignet sind darüber hinaus auch die in der WO 99/32427 offenbarten, im folgenden nochmals aufgelisteten Cyclohexan-1,2-dicarbonsäureester:
Cyclohexan-1,2-dicarbonsäuredi(isononyl)ester, erhältlich durch Hydrierung eines Di(isononyl)phthalats mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0 basierend auf Isobuten;
ein 1,2-Di-C9-Ester der Cyclohexandicarbonsäure, erhältlich durch Hydrierung eines Di(nonyl)phthalats mit der CAS Nr. 68515-46-8.

Der Inhalt der WO 99/32427, der sich u.a. auf diese vorstehend aufgelisteten Verbindungen und die Herstellung von Cyclohexanpolycarbonsäuren unter Verwendung spezieller Makroporen aufweisender Katalysatoren bezieht, wird durch Bezugnahme vollständig in die vorliegende Anmeldung einbezogen.

Des weiteren sind auch die Hydrierungsprodukte der kommerziell erhältlichen Benzolcarbonsäureester mit den Handelsnamen Jayflex DINP (CAS Nr. 68515-48-0), Jayflex DIDP (CAS Nr. 68515-49-1), Palatinol 9-P, Vestinol 9 (CAS Nr. 28553-12-0), Palatinol N (CAS Nr. 28553-12-0), Jayflex DIOP (CAS Nr. 27554-26-3), Witamol 110 (CAS Nr. 90193-91-2) und Unimoll BB (CAS Nr. 85-68-7) als geeignet im Sinne der vorliegenden Erfindung zu bewerten.

In Abhängigkeit von dem speziellen Anwendungsgebiet sind Dialkylester der 1,2-Cyclohexandicarbonsäure mit verschiedenen Alkylgruppen R besonders bevorzugt. In Klebstoffen werden 1,2-Cyclohexandicarbonsäureestern ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester, eingesetzt, wobei die 1,2-Cyclohexandicarbonsäureester bevorzugt Diisobutylreste aufweisen.

Die Herstellung der 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester erfolgt bevorzugt gemäß dem in WO 99/32427 offenbarten Verfahren. Dieses Verfahren umfasst die Hydrierung einer Benzolpolycarbonsäure oder eines Derivats davon oder eines Gemisches aus zwei oder mehr davon durch Inkontaktbringen der Benzolpolycarbonsäure oder des Derivats davon oder des Gemischs aus zwei oder mehr davon mit einem Wasserstoff enthaltenden Gas in Gegenwart eines Katalysators der als Aktivmetall mindestens ein Metall der VIII. Nebengruppe des Periodensystems allein oder zusammen mit mindestens einem Metall der I. oder VII. Nebengruppe des Periodensystems, aufgebracht auf einem Träger, umfasst, wobei der Träger Makroporen aufweist.

In einer bevorzugten Ausführungsform weist der Träger einen mittleren Porendurchmesser von mindestens 50 nm und eine BET-Oberfläche von höchstens 30 m²/g auf und die Menge des Aktivmetalls beträgt 0,01 bis 30 Gew.-%, bezogen auf das Gesamtgewicht des Katalysators.

In einer weiteren Ausführungsform wird ein Katalysator eingesetzt, worin die Menge des Aktivmetalls 0,01 bis 30 Gew.-%, bezogen auf das Gesamtgewicht des Katalysators, beträgt und 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert.

In einer weiteren Ausführungsform weist der Katalysator 0,01 bis 30 Gew.-%, bezogen auf das Gesamtgewicht des Katalysators, eines Aktivmetalls auf, aufgebracht auf einem Träger, wobei der Träger einen mittleren Porenduchmesser von mindestens 0,1 µm und eine BET-Oberfläche von höchstens 15 m²/g aufweist. Als Träger können prinzipiell alle Träger eingesetzt werden, die Makroporen aufweisen, d.h. Träger, die ausschließlich Makroporen aufweisen, sowie solche, die neben Makroporen auch Meso- und/oder Mikroporen enthalten.

Als Aktivmetall können prinzipiell alle Metalle der VIII. Nebengruppe des Periodensystems eingesetzt werden. Bevorzugt werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe "Makroporen" und "Mesoporen" werden im Rahmen der vorliegenden Anmeldung so verwendet, wie sie in Pure Appl. Chem., 45 S. 79 (1976) definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen 0,01 bis 30 Gew.-%, bevorzugt 0,01 bis 5 Gew.-%, besonders bevorzugt 0,1 bis 5 Gew.%, jeweils bezogen auf das Gesamtgewicht des verwendeten Katalysators.

Der in WO 99/32427 verwendete Begriff "Benzolpolycarbonsäure oder eines Derivats davon" umfasst alle Benzolpolycarbonsäuren an sich, zum Beispiel Phthalsäure, Isophthalsäure, Terephthalsäure, Trimellitsäure, Trimesinsäure, Hemimellitsäure und Pyrromellitsäure und Derivate davon, wobei insbesondere Mono-, Di-, Tri- und Tetraester, insbesondere Alkylester, und Anhydride zu nennen sind.

Die erfindungsgemäß eingesetzten 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester werden im Allgemeinen durch Umsetzung von Benzolpolycarbonsäuren mit den den Alkylgruppen der Ester entsprechenden Alkoholen hergestellt. Geeignete Reaktionsbedingungen zur Umsetzung der Benzolpolycarbonsäuren mit den entsprechenden Alkoholen sind dem Fachmann bekannt.

### 1,2-Cyclohexandicarbonsäureester sind nach dem folgenden Verfahren herstellbar

a) Veresterung von Phthalsäure
   mit einem oder mehreren Alkoholen der Formel

   R-OH

   worin
   R Isobutyl, 2-Ethylhexyl oder Isononyl bedeutet,
   wobei ein Phthalsäureester der Formel III erhalten wird 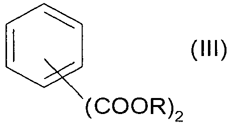
b) Hydrierung des Phthalsäureesters der Formel III zu einem entsprechenden 1,2-Cyclohexandicarbonsäureester.

Eine bevorzugte Ausführungsform der Hydrierung des Phthalsäureesters der Formel III (Schritt b)) ist vorstehend erwähnt.

Als Benzolpolycarbonsäuren wird Phthalsäure eingesetzt. Phthalsäure ist kommerziell erhältlich.

Als Alkohole werden bevorzugt die den Resten R = Isobutyl, 2-Ethylhexyl oder Isononyl der erfindungsgemäß eingesetzten 1,2-Cyclohexandicarbonsäureester entsprechenden Alkohole eingesetzt. Es werden somit Isobutanol, 2-Ethylhexanol oder Isononanol (C9-Alkohol) eingesetzt. Bei den zur Veresterung mit Phthalsäure eingesetzten Alkoholen kann es sich im Falle von Isononanol um die entsprechenden einzelnen Isomere des C9-Alkohols oder um Gemische verschiedener C9-Alkohole mit isomeren Alkylresten mit derselben Zahl von Kohlenstoffatomen handeln. Bei Isobutanol und 2-Ethylhexanol handelt es sich jeweils um einzele Isomere, d.h. um Alkohole mit einem Isobutyl- oder 2-Ethylhexylrest.

Die zur Umsetzung mit Phthalsäure geeigneten Alkohole oder Alkoholgemische können nach allen dem Fachmann bekannten Verfahren hergestellt werden. Geeignete Verfahren zur Herstellung von Alkoholen oder Verfahrensschritte, die bei der Herstellung von Alkoholen angewendet werden, sind zum Beispiel:
- Hydroformylierung mit anschließender Hydrierung der gebildeten Aldehyde, zum Beispiel wie in WO 92/13818, DE-A 2 009 505, DE-A 199 24 339, EP-A 1 113 034, WO 00/63151, WO 99/25668, JP-A 1 160 928, JP-A 03 083 935, JP-A 2000/053803, EP-A 0 278 407, EP-A 1 178 029, FR-A 1 304 144, JP-A 30 44 340, JP-A 30 44 341, JP-A 30 44 342, JP-A 0 40 36 251, GB-A 721,540, DE-A 195 304 14, JP-A 2001/049029, US 2,781,396, US 3,094,564, FR-A 1 324 873, JP-A 0 816 9854, US 3,153,673, US 3,127,451, US 1,828,344, WO 2003/66642, WO 2003/18912, EP-A 0 424 767, WO 2002/68369, EP-A 0 366 089, JP-A 2001/002829, DE-A 100 35 617, DE-A 199 55 593, WO 2002/00580, EP-A 0 643 031, US 2,876,264, JP-A 2000/319444 und DE-A 100 32 580 offenbart;
- Hydrierung von Aldolprodukten, zum Beispiel wie in DE-A 102 51 311, JP-A 05 194 761, US 3,272, 873, DE-A 3 151 086, JP-A 2001/322959, WO 98/03462 und EP-A 0 603 630 offenbart;
- Hydratisierung von Alkenen, zum Beispiel wie in US 5,136,108, EP-A 0 325 144, EP-A 0 325 143, DE-A 100 50 627, US 4,982,022, GB-A 2,187,741, DE-A 36 28 008, US 3,277,191, JP-A 2000/191 566, DE-A 854 377, DE-A 38 01 275, DE-A 39 25 217, JP-A 06 321 828, JP-A 02 088 536, JP-A 06 287 156, JP-A 06 287 155, JP-A 54 141 712, JP-A 08 283 186, JP-A 09 263 558 und US 4,684,751 offenbart.
- Hydrierung von Carbonsäuren und Carbonsäureestern, insbesondere Fettsäuren und Fettsäureestern, zum Beispiel wie in US 5,463,143, US 5,475,159, WO 94/10112, CA 2,314,690, WO 94/06738, JP-A 06 065 125 und US 3,361,832 offenbart.
- Hydrierung von ungesättigten Alkoholen oder von Carbonylverbindungen, zum Beispiel wie in EP-A 0 394 842, DE-A 1 269 605, WO 88/05767, FR-A 1,595,013, EP-A 0 326 674, BE-A 756 877, BE-A 757 561, DE-A 1 277 232, FR-A 1,499,041 und DE-A 1 276 620 offenbart;
- Hydrierung von Epoxiden, zum Beispiel wie in FR-A 1,508,939, GB-A 879 803 und DE-A 1 078 106 offenbart;
- Verfahren umfassend einen Telomerisationsschritt, zum Beispiel wie in EP-A 0 330 999, DE-A 1 138 751, US 5,908,807, NE-6,603,884 und US 3,091,628 offenbart,
- Verfahren umfassend einen Isomerisierungsschritt, zum Beispiel wie in DE-A 42 28 887 offenbart;
- Hydrolyse von Sulfaten, zum Beispiel wie in GB-A 1,165,309 offenbart;
- Umsetzung von Dienen mit Aminen, zum Beispiel wie in DE-A 44 31 528 offenbart;
- Enzymatische Herstellung von Alkoholen, zum Beispiel wie in WO 93/24644 offenbart;
- Selektive Hydrierung von Dienen, zum Beispiel wie in US 3,203,998, DE-A 21 41 186, GB-A 2,093,025, JP-A 02 129 24, JP-A 1 122 8468, DE-A 195 44 133, WO 94/00410, GB-A 2,260,136, DE-A 44 10 746 und JP-A 08 176 036 offenbart;
- Herstellung von Alkoholen aus Nitrilen, zum Beispiel wie in EP-A 0 271 092 offenbart;
- Herstellung von Alkoholen durch Umsetzung von Alkinen, zum Beispiel wie in RU 205 9597-C1 offenbart; und
- Hydrogenolyse von substituierten Tetrahydropyranen, zum Beispiel wie in GB 1,320,188 offenbart.

Dem Fachmann sind weitere Verfahren zur Herstellung von Alkoholen bekannt, die ebenfalls zur Herstellung von zur Veresterung mit Phthalsäure geeigneten Alkoholen oder Alkoholgemischen eingesetzt werden können. Eingesetzte Alkohole sind - wie vorstehend erwähnt - Isobutanol, 2-Ethylhexanol und Isononanol. Insbesondere 2-Ethylhexanol und Isononanol werden besonders bevorzugt durch katalytische Hydroformylierung (auch als Oxoreaktion bezeichnet) von Olefinen und anschließende Hydrierung der gebildeten Aldehyde hergestellt. Geeignete Hydroformylierungsverfahren sind dem Fachmann bekannt und sind in den vorstehend genannten Dokumenten offenbart. Die in den genannten Dokumenten offenbarten Alkohole und Alkoholgemische können mit Phthalsäure zu den gewünschten 1,2-Cyclohexandicarbonsäureestern bzw. -estergemischen umgesetzt werden.

Mischungen enthaltend C₈-Alkohole und deren Herstellungsverfahren sind zum Beispiel in GB-A 721 540 offenbart, worin ein Verfahren zur Herstellung von Isooctylalkoholen ausgehend von Heptenen mittels Hydroformylierung und anschließender Hydrierung beschrieben wird. Weitere Dokumente, die die Herstellung von C₇-Alkoholen bzw. diese Alkohle enthaltenden Mischungen offenbaren, sind DE-A 195 30 414, JP-A 2001/49029, US 2,781,396, US 3,094,564, FR-A 1,324,873, JP-A 08 169 854, US 3,153,673, US 3,127,451 und US 1,828,344.

C₉-Alkohole bzw. Mischungen enthaltend C₉-Alkohole werden bevorzugt durch Dimerisierung von Butenen, Hydroformylierung der erhaltenen Octene und anschließende Hydrierung des erhaltenen C₉-Aldehyds hergestellt.

Geeignete Verfahren und C₉-Alkohle enthaltende Mischungen sind zum Beispiel in WO 92/13818, DE-A 20 09 505, DE-A 199 24 339, EP-A 1 113 034, WO 2000/63151, WO 99/25668, JP-A 1 160 928, JP-A 03 083 935, JP-A 2000/053803, EP-A 0 278 407 und EP-A 1 178 029 offenbart.

Es ist möglich, dass die Alkylgruppen des Cyclohexandiisononylesters zwar die gleiche Kohlenstoffatomanzahl aufweisen aber unterschiedliche Verzweigungen aufweisen und somit Isomerengemische bilden. Der Anteil der verschiedenen Isomeren der Alkylgruppen ergibt sich im Allgemeinen aus der Zusammensetzung der C9-Alkohole, die zur Veresterung der Phthalsäure eingesetzt werden, die nach Veresterung zu den entsprechenden Cyclohexandicarbonsäureestern hydriert werden. Geeignete Alkoholmischungen sind vorstehend bereits genannt. Im Sinne der vorliegenden Anmeldung sind somit unter verzweigten Alkylresten mit einer bestimmten Anzahl von Kohlenstoffatomen neben den jeweils einzelnen Isomeren Isomerengemische zu verstehen, deren Zusammensetzung sich - wie vorstehend erwähnt - aus der Zusammensetzung der zur Veresterung der Phthalsäure eingesetzten Alkohole ergibt. Bei dem Isobutylrest bzw. dem 2-Ethylhexylrest, der gemäß der vorliegenden Anmeldung in dem erfindungsgemäß eingesetzten 1,2-Diisobutylcyclohexandicarbonsäureester bzw. 1,2-Di-(2-ethylhexyl)-cyclohexandicarbonsäureester eingesetzt wird, handelt es sich - wie vorstehend erwähnt - jeweils um definierte Reste, nicht - wie im Falle des Isononylrests möglich - um Isomerengemische.

Die Herstellung des vorstehend genannten Isobutanols und die Herstellung des vorstehend genannten 2-Ethylhexanols sind dem Fachmann bekannt.

Handelt es sich bei dem Alkylresten R der 1,2-Cyclohexandicarbonsäureester um einen Isononylrest, werden bevorzugt Isononanole (C9-Alkohole) eingesetzt, die Verzweigungsgrade (ISO-Index) von im Allgemeinen 0,10 bis 4, bevorzugt 0,5 bis 3, besonders bevorzugt 0,8 bis 2 und insbesondere bei 1 bis 1,5 aufweisen, d.h. im Allgemeinen handelt es sich bei den jeweiligen Alkoholen um Gemische verschiedener Isomere. Ganz besonders bevorzugt werden C9-Alkoholgemische mit einem ISO-Index vom 1 bis 1,5, insbesondere Nonanolgemische mit einem ISO-Index von 1,25 bzw. 1,6 eingesetzt.
Der ISO-Index ist eine dimensionslose Größe, die mittels Gaschromatographie bestimmt wurde.

| | | |
|---|---|---|
| Methode: | Kapillar GC | |
| Apparatur: | Kapillar Gaschromatograph mit Autosampler, Split/Splitless-Injektionssystem und Flammenionisationsdetektor (FID) | |
| Chemikalien: | - MSTFA (N-Methyl-N-trimethylsilyltrifluoracetamid) | |
| | - Vergleiche zur Bestimmung der Retentionszeiten | |
| Probenvorbereitung: | 3 Tropfen der Probe werden in 1 ml MSTFA und für 60 Minuten bei 80°C gehalten | |
| GC Bedingungen: | Kapillarsäule: | Ultra-1 |
| | - Länge: | 50 m |
| | - Innendurchmesser: | 0,25 mm |
| | - Filmdicke: | 0,1 Mikrometer |
| | Trägergas: | Helium |
| | Säulenvordruck: | 200 psi constant |
| | Split: | 80 ml/min |
| | Septumspülung: | 3 ml/min |
| | Ofentemperatur: | 120 °C, 25 min isotherm |
| | Injektortemperatur: | 250°C |
| | Detektortemperatur: | 250 °C (FID) |
| | Injektionsvolumen: | 0,5 Mikroliter |
| | | |
| Berechnung | Die Vorgehensweise bei der Berechnung des Isoindex wird in der folgenden Tabelle ersichtlich: | |

| **Komponente** | **Name** | **Verzweigung** | **Anteil in Fl.%** | **Index** |
|---|---|---|---|---|
| 1 | 2--Ethyl-2-methylhexanol-1 | 2 | 1,00 | 0,0200 |
| 2 | 2-Ethyl-4-methylhexanol-1 | 2 | 1,00 | 0,0200 |
| 3 | 2-Ethyl-4-methylhexanol-1 | 2 | 1,00 | 0,0200 |
| 4 | 2-Propyl-3-methylpentanol-1 | 2 | 1,00 | 0,0200 |
| 5 | 2-Propyl-hexanol-1 | 1 | 1,00 | 0,0100 |
| 6 | 2,5-Dimethylheptanol-1 | 2 | 1,00 | 0,0200 |
| 7 | 2,3-Dimethylheptanol-1 | 2 | 1,00 | 0,0200 |
| 8 | 2,3,4-Trimethylhexanol-1 | 3 | 1,00 | 0,0300 |
| 9 | 2-Ethylheptanol-1 | 1 | 1,00 | 0,0100 |
| 10 | 3-Ethyl-4-methylhexanol-1 | 2 | 82,00 | 1,6400 |
| 11 | 3-Ethylheptanol-1 | 1 | 1,00 | 0,0100 |
| 12 | 2-Methyloktanol-1 | 1 | 1,00 | 0,0100 |
| 13 | 4,5-Dimethylheptanol-1 | 2 | 1,00 | 0,0200 |
| 14 | 4,5-Dimethylheptanol-1 | 2 | 1,00 | 0,0200 |
| 15 | 4-Methyloktanol-1 | 1 | 1,00 | 0,0100 |
| 15a | 7-Methyloktanol-1 | 1 | 1,00 | 0,0000 |
| 16 | 6-Methyloktanol-1 | 1 | 1,00 | 0,0100 |
| 17 | Nonanol-1 | 0 | 1,00 | 0,0000 |
| | | **Summe:** | **99,00** | 1,9000 |
| | Unbekannte Komponente | 2 | 1,00 | 0,0200 |
| | | | **Isoindex:** | **1,9200** |

Der Isoindex berechnet sich somit aus dem Verzweigungsgrad der in dem Alkoholgemisch enthaltenen Komponenten und der Menge der entsprechenden Komponenten (ermittelt mittels Gaschromatographie).

Die Isononanole werden gemäß den vorstehend genannten Verfahren hergestellt. Es wird besonders bevorzugt ein Nonanol-Gemisch eingesetzt, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind. Unter sonstigen Komponenten sind im Allgemeinen Nonanole mit mehr als drei Verzweigungen, Decanole oder Octanole zu verstehen, wobei die Summe der genannten Komponenten 100 Gew.-% ergibt.

Eine besonders bevorzugte Ausführungsform eines Nonanol-Gemisches, das zur Herstellung von bevorzugt verwendeten Cyclohexanpolycarbonsäurederivaten eingesetzt wird, weist die folgende Zusammensetzung auf:
- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
- 0,38 bis 1,38 Gew.-%, bevorzugt 0,48 bis 1,28 Gew.-%, besonders bevorzugt 0,58 bis 1,18 Gew.-% 2,6 Dimethylheptanol;
- 2,78 bis 4,78 Gew.-%, bevorzugt 2,98 bis 4,58 Gew.-%, besonders bevorzugt 3,28 bis 4,28 Gew.-% 3,5-Dimethylheptanol;
- 6,30 bis 16,30 Gew.-%, bevorzugt 7,30 bis 15,30 Gew.-%, besonders bevorzugt 8,30 bis 14,30 Gew.-% 3,6-Dimethylheptanol;
- 5,74 bis 11,74 Gew.-%, bevorzugt 6,24 bis 11,24 Gew.-%, besonders bevorzugt 6,74 bis 10,74 Gew.-% 4,6-Dimethylheptanol;
- 1,64 bis 3,64 Gew.-%, bevorzugt 1,84 bis 3,44 Gew.-%, besonders bevorzugt 2,14 bis 3,14 Gew.-% 3,4,5-Trimethylhexanol;
- 1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew.-% 4-Methyloctanol;
- 0,98 bis 2,98 Gew.-%, bevorzugt 1,18 bis 2,78 Gew.-%, besonders bevorzugt 1,48 bis 2,48 Gew.-% 5-Methyloctaonol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew.-% 6-Methyloctanol;
- 0,1 bis 3 Gew.-%, bevorzugt 0,2 bis 2 Gew.-%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen; wobei die Gesamtsumme der genannten Komponenten 100 Gew.-% ergibt.

Besonders bevorzugt sind erfindungsgemäße Tapeten, Bodenbeläge aus Linoleum, Gummi oder Textil, Parkett oder Isolierglasfenster, wobei der Klebstoff einen Diisononylcyclohexandicarbonsäureester enthält, wobei zu dessen Herstellung ein Nonanol-Gemisch eingesetzt wird, welches die folgende Zusammensetzung aufweist:
- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
- 0,38 bis 1,38 Gew.-%, bevorzugt 0,48 bis 1,28 Gew.-%, besonders bevorzugt 0,58 bis 1,18 Gew.-% 2,6 Dimethylheptanol;
- 2,78 bis 4,78 Gew. -%, bevorzugt 2,98 bis 4,58 Gew. -%, besonders bevorzugt 3,28 bis 4,28 Gew.-% 3,5-Dimethylheptanol;
- 6,30 bis 16,30 Gew. -%, bevorzugt 7,30 bis 15,30 Gew. -%, besonders bevorzugt 8,30 bis 14,30 Gew.-% 3,6-Dimethylheptanol;
- 5,74 bis 11,74 Gew. -%, bevorzugt 6,24 bis 11,24 Gew. -%, besonders bevorzugt 6,74 bis 10,74 Gew.-% 4,6-Dimethylheptanol;
- 1,64 bis 3,64 Gew. -%, bevorzugt 1,84 bis 3,44 Gew. -%, besonders bevorzugt 2,14 bis 3,14 Gew.-% 3,4,5-Trimethylhexanol;
- 1,47 bis 5,47 Gew. -%, bevorzugt 1,97 bis 4,97 Gew. -%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew. -%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew. -% 4-Methyloctanol;
- 0,98 bis 2,98 Gew.-%, bevorzugt 1,18 bis 2,78 Gew. -%, besonders bevorzugt 1,48 bis 2,48 Gew.-% 5-Methyloctaonol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew. -% 6-Methyloctanol;
- 0,1 bis 3 Gew. -%, bevorzugt 0,2 bis 2 Gew. -%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen;
wobei die Gesamtsumme der genannten Komponenten 100 Gew.-% ergibt.

Eine weitere besonders bevorzugte Ausführungsform eines Nonanol-Gemisches, das zur Herstellung von bevorzugt verwendeten Cyclohexanpolycarbonsäurederivaten eingesetzt wird, weist die folgende Zusammensetzung auf:
- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.-% 2-Methyloctanol;
- 5,7 bis 11,7 Gew.-%, bevorzugt 6,3 bis 11,3 Gew.-%, besonders bevorzugt 6,7 bis 10,7 Gew.-% 3-Ethylheptanol;
- 1,9 bis 3,9 Gew.-%, bevorzugt 2,1 bis 3,7 Gew.-%, besonders bevorzugt 2,4 bis 3,4 Gew.-% 2-Ethylheptanol;
- 1,7 bis 3,7 Gew.-%, bevorzugt 1,9 bis 3,5 Gew.-%, besonders bevorzugt 2,2 bis 3,2 Gew.-% 2-Propylhexanol;
- 3,2 bis 9,2 Gew.-%, bevorzugt 3,7 bis 8,7 Gew.-%, besonders bevorzugt 4,2 bis 8,2 Gew.-% 3,5-Dimethylheptanol;
- 6,0 bis 16,0 Gew.-%, bevorzugt 7,0 bis 15,0 Gew.-%, besonders bevorzugt 8,0 bis 14,0 Gew.-% 2,5-Dimethylheptanol;
- 1,8 bis 3,8 Gew.-%, bevorzugt 2,0 bis 3,6 Gew.-%, besonders bevorzugt 2,3 bis 3,3 Gew.-% 2,3-Dimethylheptanol;
- 0,6 bis 2,6 Gew.-%, bevorzugt 0,8 bis 2,4 Gew.-%, besonders bevorzugt 1,1 bis 2,1 Gew.-% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.-% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
wobei die Gesamtsumme der genannten Komponenten 100 Gew.-% ergibt.

Besonders bevorzugt sind erfindungsgemäße Tapeten, Bodenbeläge aus Linoleum, Gummi oder Textil, Parkett oder Isolierglasfenster, wobei der Klebstoff ein Diisononylcyclohexandicarbonsäureester enthält, wobei zu dessen Herstellung ein Nonanol-Gemisch eingesetzt wird, welches die folgende Zusammensetzung aufweist:
- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.-% 2-Methyloctanol;
- 5,7 bis 11,7 Gew.-%, bevorzugt 6,3 bis 11,3 Gew.-%, besonders bevorzugt 6,7 bis 10,7 Gew. -% 3-Ethylheptanol;
- 1,9 bis 3,9 Gew.-%, bevorzugt 2,1 bis 3,7 Gew.-%, besonders bevorzugt 2,4 bis 3,4 Gew.-% 2-Ethylheptanol;
- 1,7 bis 3,7 Gew.-%, bevorzugt 1,9 bis 3,5 Gew.-%, besonders bevorzugt 2,2 bis 3,2 Gew.-% 2-Propylhexanol;
- 3,2 bis 9,2 Gew.-%, bevorzugt 3,7 bis 8,7 Gew.-%, besonders bevorzugt 4,2 bis 8,2 Gew.-% 3,5-Dimethylheptanol;
- 6,0 bis 16,0 Gew. -%, bevorzugt 7,0 bis 15,0 Gew.-%, besonders bevorzugt 8,0 bis 14,0 Gew. -% 2,5-Dimethylheptanol;
- 1,8 bis 3,8 Gew.-%, bevorzugt 2,0 bis 3,6 Gew.-%, besonders bevorzugt ,3 bis 3,3 Gew.-% 2,3-Dimethylheptanol;
- 0,6 bis 2,6 Gew.-%, bevorzugt 0,8 bis 2,4 Gew.-%, besonders bevorzugt 1,1 bis 2,1 Gew.-% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.-% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
wobei die Gesamtsumme der genannten Komponenten 100 Gew. -% ergibt.

### 1,2-Cyclohexandicarbonsäureester sind herstellbar durch ein Verfahren umfassend die Schritte

a) Veresterung von Phthalsäure
   mit einem oder mehreren Alkoholen der Formel

   R'-OH

   worin
   R' Isobutyl, 2-Ethylhexyl oder Isononyl bedeutet,
   wobei - im Falle von Isononyl - die Alkylreste R' Verzweigungsgrade von 0,1 bis 4, bevorzugt 0,5 bis 3, besonders bevorzugt 0,8 bis 2, ganz besonders bevorzugt 1 bis 1,5 aufweisen (ISO-Index),
   wobei ein Phthalsäureester der Formel III' erhalten wird 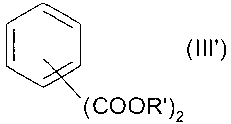
b) Hydrierung des Phthalsäureesters der Formel III' zu einem entsprechenden Cyclohexanpolycarbonsäureester.

Bevorzugte Alkohole R'-OH, insbesondere Nonanolgemische, sind die vorstehend genannten Alkohole und Alkoholgemische.

Die erfindungsgemäßen 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester sind besonders gut als oder in Hilfsmitteln geeignet.

Die 1,2-Cyclohexandicarbonsäureester sind insbesondere zur Verwendung in oberflächenaktiven Zusammensetzungen ausgewählt aus der Gruppe bestehend aus Fließ-, Filmbildehilfen, Entschäumern, Schaumverhütungsmitteln, Benetzungsmitteln, Koaleszenzmitteln und Emulgatoren geeignet.

Ein weiterer Gegenstand der vorliegenden Anmeldung ist ein Klebstoff enthaltend mindestens ein 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester.

Diese Klebstoffe können vielfältig eingesetzt werden. Die die offenbarten 1,2-Cyclohexandicarbonsäureester enthaltenden Klebstoffe können z.B. in den Bereichen Papier- und Verpackung, Holz, im Baubereich, in Haushalt, Hobby und Büro, in der Automobilindustrie, in der Medizin, in der Elektronik, in der Schuhproduktion, sowie in Klebebändern in den genannten Anwendungsbereichen eingesetzt werden. In einer bevorzugten Ausführungsform werden die 1,2-Cyclohexandicarbonsäureester enthaltenden Klebstoffe in der Medizin eingesetzt. Dies ist vorteilhaft, da die erfindungsgemäß eingesetzten 1,2-Cyclohexandicarbonsäureester nicht allergieauslösend wirken.

In den genannten Bereichen können erfindungsgemäß die 1,2-Cyclohexandicarbonsäureester in verschiedenen Klebstoffarten für verschiedene Anwendungen eingesetzt werden. Im Folgenden werden die genannten Bereiche sowie die geeigneten Klebstoffe näher erläutert:

### Papier- und Verpackung

Die die 1,2-Cyclohexandicarbonsäureester enthaltenden Klebstoffe werden zur Herstellung von undurchlässigen Verpackungsmaterialien wie Verbundfolien oder zum hermetischen Verschluss von Verpackungen (z.B. Kaffeeverpackungen) für die moderne Form von Vertrieb, Selbstbedienung, Fertiggerichten und Tiefkühlkost eingesetzt.

Tief- und hochtemperaturbeständige Klebstoffe ermöglichen die Herstellung von Tiefkühl- und Mikrowellenverpackungen. Dabei müssen die Klebstoffe zur Herstellung von Lebensmittelverpackungen den strengen Vorschriften des Lebensmittelgesetzes genügen.

Weitere Einsatzgebiete der die 1,2-Cyclohexandicarbonsäureester enthaltenden Klebstoffe sind die Herstellung von Zigaretten und Etiketten sowie zur Herstellung von Papier und zur Produktion von Zeitungen und zum Binden von Büchern, Katalogen etc. eingesetzt werden, sowie als Klebstoffe für Briefmarken und Verschlüsse von Briefumschlägen.

### Holz

Besonders zur Anwendung im Holzbereich geeignete Klebstoffe, die die 1,2-Cyclohexandicarbonsäureester enthalten, sind Polykondensations-, Dispersions- und Schmelzklebstoffe. Die Zusammensetzung dieser Klebstoffe ist dem Fachmann bekannt.

### Baubereich

Die die erfindungsgemäß verwendeten 1,2-Cyclohexandicarbonsäureester enthaltenden Klebstoffe werden in Tapeten, Bodenbelägen aus Linoleum, Gummi oder Textil, Parkett, oder Isolierglasfenstern eingesetzt.

### Haushalt, Hobby und Büro

Die die 1,2-Cyclohexandicarbonsäureester enthaltenden Klebstoffe können in Allesklebern, Klebestiften, Papier- und Bastelklebstoffen, Kontaktklebstoffen, Sekundenklebern, sowie Klebstoffen für Kunststoff- und Pappen, Karton, Fotos und Etiketten eingesetzt werden. Sie zeichnen sich durch einfaches, sauberes, schnelles und durch den Verzicht auf Lösungsmittel umweltfreundliches Kleben aus. Des Weiteren können die Klebstoffe als Modellbauklebstoffe, Montageklebstoffe und 2-Komponenten-Klebstoffe eingesetzt werden. Dabei sind auf Wasser basierende oder lösungsmittelfreie Klebstoffsysteme bevorzugt.

### Automobilindustrie

Die die 1,2-Cyclohexandicarbonsäureester enthaltenden Klebstoffe sind zur Anwendung in der Automobilindustrie geeignet. Sie werden z.B. in Klebstoffen auf Basis von Polyurethan oder in 2-Komponenten-Klebstoffen eingesetzt. Die Zusammensetzung solcher Klebstoffe ist dem Fachmann bekannt.

### Medizin

Die die 1,2-Cyclohexandicarbonsäureester enthaltenden Klebstoffe können z.B. in Pflastern, zum Kleben von Gelenkprothesen, in der Zahnmedizin zum Kleben von Brücken, Kronen, Verblendschalen und Inlays und zur Produktion jeder Art von Hygieneartikeln wie Windeln, Einlagen, OP-Tüchern und Papiertaschentücher eingesetzt werden. Sie finden ebenfalls Verwendung bei der Konfektionierung von Medikamenten in Blisterverpackungen, um die Tabletten vor Feuchtigkeitseinflüssen, Schmutz und Bakterien zu schützen.

### Elektronik

Die 1,2-Cyclohexandicarbonsäureester werden z.B. in photoinitiierten Kleb-, Dicht- und Vergußmassen für den Chipverguß, in der Chip-on-board-Technologie, für Chip-Encapsulation sowie als Flip-Chip-Underfiller eingesetzt.

### Schuhproduktion

Für Klebungen im Schuhinnenbereich dienen Natur- oder Synthese-Kautschuk-Klebstoffe, Dispersionsklebstoffe auf Grundlage von Kunststoffpolymeren sowie Klebstoffe aus wässriger Basis aus Stärke, Dextrin und Cellulosederivaten. Alle diese Klebstoffe werden vornehmlich nach dem Naßklebeverfahren verarbeitet. Die genannten Klebstoffe enthalten 1,2-Cyclohexandicarbonsäureester. Geeignete Zusammensetzungen der Klebstoffe sind dem Fachmann bekannt.

Schmelzklebstoffe auf Basis von Ethylen-Vinylacetat-Copolymeren werden zum Kaschieren, Polyamid-Klebstoffe zum Buggen verwendet. Diese Klebstoffe enthalten ebenfalls erfindungsgemäß 1,2-Cyclohexandicarbonsäureester. Geeignete Zusammensetzungen der Klebstoffe sind dem Fachmann bekannt.

Die Verbindung zwischen Schuhoberteil und Brandsohle, "Zwicken" genannt, kann mit Polyamid- oder Polyester-Schmelzklebstoffen, die ebenfalls die die erfindungsgemäß verwendeten 1,2-Cyclohexandicarbonsäureester enthalten, ausgeführt werden.

Das Kleben der Laufsohlen auf den Schuhschaft kann mit Lösemittel- oder Dispersions-Klebstoffen auf Polychloropren- oder Polyurethan-Basis, die ebenfalls die die erfindungsgemäß verwendeten 1,2-Cyclohexandicarbonsäureester enthalten, erfolgen.

### Klebebänder

Die die 1,2-Cyclohexandicarbonsäureester enthaltenden Klebstoffe können des Weiteren in Klebebändern eingesetzt werden.

Selbstklebebänder werden als Problemlösungskonzepte für die Industrie, für andere gewerbliche Abnehmer sowie für den privaten Bedarf hergestellt. Der Markt verfügt über eine Vielzahl modernster Klebetechnologien und bietet individuelle Problemlösungen für alle wichtigen Anwendungen mit Schwerpunkten in der Verpackungs-, Elektro-, Verkehrsmittel- und Papierindustrie.

Je nach Einsatz der Klebebänder werden unterschiedliche Ansprüche an die wesentlichen Produkteigenschaften wie Klebkraft, Temperaturbeständigkeit, mechanische Belastbarkeit und Zugfestigkeit gestellt. Beispielsweise müssen Klebebänder zur Abdeckung bei industriellen Lackiervorgängen hohe Temperaturen aushalten und später wieder rückstandsfrei entfernt werden können. Bei Ummantelungen und Bündelungen kommt es jedoch auf hohe Festigkeit und geringe Dehnung an. Hohe sofortige Klebkraft ist dagegen beim Rollenwechsel in der Papierindustrie notwendig. Außerhalb der industriellen Anwendungsbereiche finden diese Produkte vor allem in der Schule, im Haushalt, im Büro oder im Do-it-yourself-Sektor Anwendung.

Die Klebstoffe können - neben den 1,2-Cyclohexandicarbonsäureestern - einen oder mehrere der folgenden Bestandteile enthalten, wobei man grundsätzlich zwischen Klebstoffen auf natürlicher und synthetischer Rohstoff-Basis unterscheidet, wobei auch Kombinationen dieser Grundstoffe möglich sind. Geeignete Rohstoffe sind dem Fachmann bekannt.

Geeignete Zusammensetzungen der Klebstoffe, insbesondere zum Einsatz in der Medizin, z.B. als Haftmittel für das Auftragen auf die Haut von Menschen oder Tieren, sind zum Beispiel in EP-A 0 928 207 offenbart.

Bevorzugt wird in den genannten Klebstoffen oder als Klebstoffe 1,2-Diisobutylcyclohexandicarbonsäureester eingesetzt.

Weiterhin eignen sich die 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester als Weichmacher für Schlagzähmodifier, bevorzugt für Schlagzähmodifier für thermoplastische Kunststoffe, zum Beispiel für Polyamid. Geeignete Mengen, in denen der mindestens eine 1,2-Cyclohexandicarbonsäureester eingesetzt wird, sind die für Weichmacher üblichen Mengen und somit dem Fachmann bekannt.

Bevorzugt werden in den Schlagzähmodifiern oder als Schlagzähmodifier 1,2-Di-(2-ethylhexyl)-cyclohexandicarbonsäureester und 1,2-Diisononylcyclohexandicarbonsäureester eingesetzt.

Des Weiteren können die 1,2-Cyclohexandicarbonsäureester ausgewählt aus der Gruppe bestehend aus 1,2-Diisobutylcyclohexandicarbonsäureester, 1,2-Di-(2-ethylhexyl)-cyclohexandicarbonsäureester und 1,2-Diisononylcyclohexandicarbonsäureester als Hilfsmittel bei der Herstellung von Waschmitteln eingesetzt werden, zum Beispiel als Stellmittel, um Waschmittelpulver rieselfähig zu halten und in einen anwendungsgerechten Zustand zu bringen. Geeignete Mengen entsprechen den Mengen von üblicherweise in Waschmitteln eingesetzten Stellmitteln und sind dem Fachmann bekannt.

Bevorzugt werden in den Waschmitteln 1,2-Di-(2-ethylhexyl)-cyclohexandicarbonsäureester und 1,2-Diisononylcyclohexandicarbonsäureester eingesetzt.

Die Herstellung der Hilfsmittel, die die 1,2-Cyclohexandicarbonsäureester enthalten, erfolgt jeweils nach dem Fachmann bekannten Verfahren.

## Patentansprüche

1. Tapeten, Bodenbeläge aus Linoleum, Gummi oder Textil, Parkett oder Isolierglasfenster enthaltend einen Klebstoff, enthaltend mindestens einen 1,2-Cyclohexandicarbonsäureester ausgewählt aus der Gruppe bestehend aus 1,2-Diisobutylcyclohexandicarbonsäureester, 1,2-Di-(2-ethylhexyl)cyclohexandicarbonsäureester und Diisononylcyclohexandicarbonsäureester.

2. Tapeten, Bodenbeläge aus Linoleum, Gummi oder Textil, Parkett oder Isolierglasfenster nach Anspruch 1, **dadurch gekennzeichnet, dass** der Klebstoff einen Diisononylcyclohexandicarbonsäureester enthält, wobei zu dessen Herstellung ein Nonanol-Gemisch eingesetzt wird, welches die folgende Zusammensetzung aufweist:
- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
- 0,38 bis 1,38 Gew.-%, bevorzugt 0,48 bis 1,28 Gew.-%, besonders bevorzugt 0,58 bis 1,18 Gew.-% 2,6 Dimethylheptanol;
- 2,78 bis 4,78 Gew. -%, bevorzugt 2,98 bis 4,58 Gew. -%, besonders bevorzugt 3,28 bis 4,28 Gew.-% 3,5-Dimethylheptanol;
- 6,30 bis 16,30 Gew. -%, bevorzugt 7,30 bis 15,30 Gew. -%, besonders bevorzugt 8,30 bis 14,30 Gew.-% 3,6-Dimethylheptanol;
- 5,74 bis 11,74 Gew. -%, bevorzugt 6,24 bis 11,24 Gew. -%, besonders bevorzugt 6,74 bis 10,74 Gew.-% 4,6-Dimethylheptanol;
- 1,64 bis 3,64 Gew. -%, bevorzugt 1,84 bis 3,44 Gew. -%, besonders bevorzugt 2,14 bis 3,14 Gew.-% 3,4,5-Trimethylhexanol;
- 1,47 bis 5,47 Gew. -%, bevorzugt 1,97 bis 4,97 Gew. -%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew. -%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew. -% 4-Methyloctanol;
- 0,98 bis 2,98 Gew.-%, bevorzugt 1,18 bis 2,78 Gew. -%, besonders bevorzugt 1,48 bis 2,48 Gew.-% 5-Methyloctaonol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew. -% 6-Methyloctanol;
- 0,1 bis 3 Gew. -%, bevorzugt 0,2 bis 2 Gew. -%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen;
wobei die Gesamtsumme der genannten Komponenten 100 Gew.-% ergibt.

3. Tapeten, Bodenbeläge aus Linoleum, Gummi oder Textil, Parkett oder Isolierglasfenster nach Anspruch 1, **dadurch gekennzeichnet, dass** der Klebstoff ein Diisononylcyclohexandicarbonsäureester enthält, wobei zu dessen Herstellung ein Nonanol-Gemisch eingesetzt wird, welches die folgende Zusammensetzung aufweist:
- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.-% 2-Methyloctanol;
- 5,7 bis 11,7 Gew.-%, bevorzugt 6,3 bis 11,3 Gew.-%, besonders bevorzugt 6,7 bis 10,7 Gew. -% 3-Ethylheptanol;
- 1,9 bis 3,9 Gew.-%, bevorzugt 2,1 bis 3,7 Gew.-%, besonders bevorzugt 2,4 bis 3,4 Gew.-% 2-Ethylheptanol;
- 1,7 bis 3,7 Gew.-%, bevorzugt 1,9 bis 3,5 Gew.-%, besonders bevorzugt 2,2 bis 3,2 Gew.-% 2-Propylhexanol;
- 3,2 bis 9,2 Gew.-%, bevorzugt 3,7 bis 8,7 Gew.-%, besonders bevorzugt 4,2 bis 8,2 Gew.-% 3,5-Dimethylheptanol;
- 6,0 bis 16,0 Gew. -%, bevorzugt 7,0 bis 15,0 Gew.-%, besonders bevorzugt 8,0 bis 14,0 Gew. -% 2,5-Dimethylheptanol;
- 1,8 bis 3,8 Gew.-%, bevorzugt 2,0 bis 3,6 Gew.-%, besonders bevorzugt ,3 bis 3,3 Gew.-% 2,3-Dimethylheptanol;
- 0,6 bis 2,6 Gew.-%, bevorzugt 0,8 bis 2,4 Gew.-%, besonders bevorzugt 1,1 bis 2,1 Gew.-% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.-% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
wobei die Gesamtsumme der genannten Komponenten 100 Gew. -% ergibt.

4. Verwendung eines Klebstoffes wie in einem der Ansprüche 1 bis 3 definiert, in Tapeten, Bodenbelägen aus Linoleum, Gummi oder Textil, Parkett oder Isolierglasfenstern.

## Claims

1. Wallpapers, floorcoverings composed of linoleum, rubber or textile, parquet or double glazing comprising an adhesive comprising at least one 1,2-cyclohexanedicarboxylic ester selected from the group consisting of diisobutyl 1,2-cyclohexanedicarboxylate, di(2-ethylhexyl) 1,2-cyclohexanedicarboxylate and diisononyl cyclohexanedicarboxylate.

2. Wallpapers, floorcoverings composed of linoleum, rubber or textile, parquet or double glazing according to Claim 1, **characterized in that** the adhesive comprises a diisononyl cyclohexanedicarboxylate prepared using a nonanol mixture having the following composition:
- from 1.73 to 3.73% by weight, preferably from 1.93 to 3.53% by weight, particularly preferably from 2.23 to 3.23% by weight, of 3-ethyl-6-methylhexanol;
- from 0.38 to 1.38% by weight, preferably from 0.48 to 1.28% by weight, particularly preferably from 0.58 to 1.18% by weight, of 2,6-dimethylheptanol;
- from 2.78 to 4.78% by weight, preferably from 2.98 to 4.58% by weight, particularly preferably from 3.28 to 4.28% by weight, of 3,5-dimethylheptanol;
- from 6.30 to 16.30% by weight, preferably from 7.30 to 15.30% by weight, particularly preferably from 8.30 to 14.30% by weight, of 3,6-dimethylheptanol;
- from 5.74 to 11.74% by weight, preferably from 6.24 to 11.24% by weight, particularly preferably from 6.74 to 10.74% by weight, of 4,6-dimethylheptanol;
- from 1.64 to 3.64% by weight, preferably from 1.84 to 3.44% by weight, particularly preferably from 2.14 to 3.14% by weight, of 3,4,5-trimethylhexanol;
- from 1.47 to 5.47% by weight, preferably from 1.97 to 4.97% by weight, particularly preferably from 2.47 to 4.47% by weight, of 3,4,5-trimethylhexanol, 3-methyl-4-ethylhexanol, and 3-ethyl-4-methylhexanol;
- from 4.00 to 10.00% by weight, preferably from 4.50 to 9.50% by weight, particularly preferably from 5.00 to 9.00% by weight, of 3,4-dimethylheptanol;
- from 0.99 to 2.99% by weight, preferably from 1.19 to 2.79% by weight, particularly preferably from 1.49 to 2.49% by weight, of 4-ethyl-5-methylhexanol and 3-ethylheptanol;
- from 2.45 to 8.45% by weight, preferably from 2.95 to 7.95% by weight, particularly preferably from 3.45 to 7.45% by weight, of 4,5-dimethylheptanol and 3-methyloctanol;
- from 1.21 to 5.21% by weight, preferably from 1.71 to 4.71% by weight, particularly preferably from 2.21 to 4.21% by weight, of 4,5-dimethylheptanol;
- from 1.55 to 5.55% by weight, preferably from 2.05 to 5.05% by weight, particularly preferably from 2.55 to 4.55% by weight, of 5,6-dimethylheptanol;
- from 1,63 to 3.63% by weight, preferably from 1.83 to 3.43% by weight, particularly preferably from 2.13 to 3.13% by weight, of 4-methyloctanol;
- from 0.98 to 2.98% by weight, preferably from 1.18 to 2.78% by weight, particularly preferably from 1.48 to 2.48% by weight, of 5-methyloctanol;
- from 0.70 to 2.70% by weight, preferably from 0.90 to 2.50% by weight, particularly preferably from 1.20 to 2.20% by weight, of 3,6,6-trimethylhexanol;
- from 1.96 to 3.96% by weight, preferably from 2.16 to 3.76% by weight, particularly preferably from 2.46 to 3.46% by weight, of 7-methyloctanol;
- from 1.24 to 3.24% by weight, preferably from 1.44 to 3.04% by weight, particularly preferably from 1.74 to 2.74% by weight, of 6-methyloctanol;
- from 0.1 to 3% by weight, preferably from 0.2 to 2% by weight, particularly preferably from 0.3 to 1% by weight, of n-nonanol;
- from 25 to 35% by weight, preferably from 28 to 33% by weight, particularly preferably from 29 to 32% by weight, of other alcohols having 9 or 10 carbon atoms;
where the entirety of the components mentioned gives 100% by weight.

3. Wallpapers, floorcoverings composed of linoleum, rubber or textile, parquet or double glazing according to Claim 1, **characterized in that** the adhesive comprises a diisononyl cyclohexanedicarboxylate prepared using a nonanol mixture having the following composition:
- from 6.0 to 16.0% by weight, preferably from 7.0 to 15.0% by weight, particularly preferably from 8.0 to 14.0% by weight, of n-nonanol;
- from 12.8 to 28.8% by weight, preferably from 14.8 to 26.8% by weight, particularly preferably from 15.8 to 25.8% by weight, of 6-methyloctanol;
- from 12.5 to 28.8% by weight, preferably from 14.5 to 26.5% by weight, particularly preferably from 15.5 to 25.5% by weight, of 4-methyloctanol;
- from 3.3 to 7.3% by weight, preferably from 3.8 to 6.8% by weight, particularly preferably from 4.3 to 6.3% by weight, of 2-methyloctanol;
- from 5.7 to 11.7% by weight, preferably from 6.3 to 11.3% by weight, particularly preferably from 6.7 to 10.7% by weight, of 3-ethylheptanol;
- from 1.9 to 3.9% by weight, preferably from 2.1 to 3.7% by weight, particularly preferably from 2.4 to 3.4% by weight, of 2-ethylheptanol;
- from 1.7 to 3.7% by weight, preferably from 1.9 to 3.5% by weight, particularly preferably from 2.2 to 3.2% by weight, of 2-propylhexanol;
- from 3.2 to 9.2% by weight, preferably from 3.7 to 8.7% by weight, particularly preferably from 4.2 to 8.2% by weight, of 3,5-dimethylheptanol;
- from 6.0 to 16.0% by weight, preferably from 7.0 to 15.0% by weight, particularly preferably from 8.0 to 14.0% by weight, of 2,5-dimethylheptanol;
- from 1.8 to 3.8% by weight, preferably from 2.0 to 3.6% by weight, particularly preferably from 2.3 to 3.3% by weight, of 2,3-dimethylheptanol;
- from 0.6 to 2.6% by weight, preferably from 0.8 to 2.4% by weight, particularly preferably from 1.1 to 2.1% by weight, of 3-ethyl-4-methylhexanol;
- from 2.0 to 4.0% by weight, preferably from 2.2 to 3.8% by weight, particularly preferably from 2.5 to 3.5% by weight, of 2-ethyl-4-methylhexanol;
- from 0.5 to 6.5% by weight, preferably from 1.5 to 6% by weight, particularly preferably from 1.5 to 5.5% by weight, of other alcohols having 9 carbon atoms;
where the entirety of the components mentioned gives 100% by weight.

4. Use of an adhesive as defined in any one of Claims 1 to 3 in wallpapers, floorcoverings composed of linoleum, rubber or textile, parquet or double glazing.

## Revendications

1. Tapis, revêtements de sol en linoléum, caoutchouc ou textile, parquet ou fenêtre vitrée isolante, contenant un adhésif, contenant au moins un ester de l'acide 1,2-cyclohexanedicarboxylique, choisi dans le groupe constitué par un ester de l'acide 1,2-diisobutylcyclohexanedicarboxylique, un ester de l'acide 1,2-di-(2-éthylhexyl)cyclohexanedicarboxylique et un ester de l'acide diisononylcyclohexanedicarboxylique.

2. Tapis, revêtements de sol en linoléum, caoutchouc ou textile, parquet ou fenêtre vitrée isolante selon la revendication 1, caractérisé(e)(s) en ce que l'adhésif contient un ester de l'acide diisononylcyclohexanedicarboxylique, un mélange de nonanols étant utilisé pour sa préparation, qui présente la composition suivants :
- 1,73 à 3,73% en poids, de préférence 1,93 à 3,53% en poids, de manière particulièrement préférée 2,23 à 3,23% en poids de 3-éthyl-6-méthylhexanol ;
- 0,38 à 1,38% en poids, de préférence 0,48 à 1,28% en poids, de manière particulièrement préférée 0,58 à 1,18% en poids de 2,6-diméthylheptanol ;
- 2,78 à 4,78% en poids, de préférence 2,98 à 4,58% en poids, de manière particulièrement préférée 3,28 à 4,28% en poids de 3,5-diméthylheptanol ;
- 6,30 à 16,30% en poids, de préférence 7,30 à 15,30% en poids, de manière particulièrement préférée 8,30 à 14,30% en poids de 3,6-diméthylheptanol ;
- 5,74 à 11,74% en poids, de préférence 6,24 à 11,24% en poids, de manière particulièrement préférée 6,74 à 10,74% en poids de 4,6-diméthylheptanol ;
- 1,64 à 3,64% en poids, de préférence 1,84 à 3,44% en poids, de manière particulièrement préférée 2,14 à 3,14% en poids de 3,4,5-triméthylhexanol ;
- 1,47 à 5,47% en poids, de préférence 1,97 à 4,97% en poids, de manière particulièrement préférée 2,47 à 4,47% en poids de 3,4,5-triméthylhexanol, de 3-méthyl-4-éthylhexanol et de 3-éthyl-4-méthylhexanol ;
- 4,00 à 10,00% en poids, de préférence 4,50 à 9,50% en poids, de manière particulièrement préférée 5,00 à 9,00% en poids de 3,4-diméthylheptanol ;
- 0,99 à 2,99% en poids, de préférence 1,19 à 2,79% en poids, de manière particulièrement préférée 1,49 à 2,49% en poids de 4-éthyl-5-méthylhexanol et de 3-éthylheptanol ;
- 2,45 à 8,45% en poids, de préférence 2,95 à 7,95% en poids, de manière particulièrement préférée 3,45 à 7,45% en poids de 4,5-diméthylheptanol et de 3-méthyloctanol ;
- 1,21 à 5,21% en poids, de préférence 1,71 à 4,71% en poids, de manière particulièrement préférée 2,21 à 4,21% en poids de 4,5-diméthylheptanol ;
- 1,55 à 5,55% en poids, de préférence 2,05 à 5,05% en poids, de manière particulièrement préférée 2,55 à 4,55% en poids de 5,6-diméthylheptanol ;
- 1,63 à 3,63% en poids, de préférence 1,83 à 3,43% en poids, de manière particulièrement préférée 2,13 à 3,13% en poids de 4-méthyloctanol ;
- 0,98 à 2,98% en poids, de préférence 1,18 à 2,78% en poids, de manière particulièrement préférée 1,48 à 2,48% en poids de 5-méthyloctaonol ;
- 0,70 à 2,70% en poids, de préférence 0,90 à 2,50% en poids, de manière particulièrement préférée 1,20 à 2,20% en poids de 3,6,6-triméthylhexanol ;
- 1,96 à 3,96% en poids, de préférence 2,16 à 3,76% en poids, de manière particulièrement préférée 2,46 à 3,46% en poids de 7-méthyloctanol ;
- 1,24 à 3,24% en poids, de préférence 1,44 à 3,04% en poids, de manière particulièrement préférée 1,74 à 2,74% en poids de 6-méthyloctanol ;
- 0,1 à 3% en poids, de préférence 0,2 à 2% en poids, de manière particulièrement préférée 0,3 à 1% en poids de n-nonanol ;
- 25 à 35% en poids, de préférence 28 à 33% en poids, de manière particulièrement préférée 29 à 32% en poids d'autres alcools comprenant 9 et 10 atomes de carbone ;
la somme totale des composants mentionnés valant 100% en poids.

3. Tapis, revêtements de sol en linoléum, caoutchouc ou textile, parquet ou fenêtre vitrée isolante selon la revendication 1, caractérisé(e)(s) en ce que l'adhésif contient un ester de l'acide diisononylcyclohexanedicarboxylique, un mélange de nonanols étant utilisé pour sa préparation, qui présente la composition suivants :
- 6,0 à 16,0% en poids, de préférence 7,0 à 15,0% en poids, de manière particulièrement préférée 8,0 à 14,0% en poids de n-nonanol ;
- 12,8 à 28,8% en poids, de préférence 14,8 à 26,8% en poids, de manière particulièrement préférée 15,8 à 25,8% en poids de 6-méthyloctanol ;
- 12,5 à 28,8% en poids, de préférence 14,5 à 26,5% en poids, de manière particulièrement préférée 15,5 à 25,5% en poids de 4-méthyloctanol ;
- 3,3 à 7,3% en poids, de préférence 3,8 à 6,8% en poids, de manière particulièrement préférée 4,3 à 6,3% en poids de 2-méthyloctanol ;
- 5,7 à 11,7% en poids, de préférence 6,3 à 11,3% en poids, de manière particulièrement préférée 6,7 à 10,7% en poids de 3-éthylheptanol ;
- 1,9 à 3,9% en poids, de préférence 2,1 à 3,7% en poids, de manière particulièrement préférée 2,4 à 3,4% en poids de 2-éthylheptanol ;
- 1,7 à 3,7% en poids, de préférence 1,9 à 3,5% en poids, de manière particulièrement préférée 2,2 à 3,2% en poids de 2-propylhexanol ;
- 3,2 à 9,2% en poids, de préférence 3,7 à 8,7% en poids, de manière particulièrement préférée 4,2 à 8,2% en poids de 3,5-diméthylheptanol ;
- 6,0 à 16,0% en poids, de préférence 7,0 à 15,0% en poids, de manière particulièrement préférée 8,0 à 14,0% en poids de 2,5-diméthylheptanol ;
- 1,8 à 3,8% en poids, de préférence 2,0 à 3,6% en poids, de manière particulièrement préférée 3 à 3,3% en poids de 2,3-diméthylheptanol ;
- 0,6 à 2,6% en poids, de préférence 0,8 à 2,4% en poids, de manière particulièrement préférée 1,1 à 2,1% en poids de 3-éthyl-4-méthylhexanol ;
- 2,0 à 4,0% en poids, de préférence 2,2 à 3,8% en poids, de manière particulièrement préférée 2,5 à 3,5% en poids de 2-éthyl-4-méthylhexanol ;
- 0,5 à 6,5% en poids, de préférence 1,5 à 6% en poids, de manière particulièrement préférée 1,5 à 5,5% en poids d'autres alcools comprenant 9 atomes de carbone ;
la somme totale des composants mentionnés valant 100% en poids.

4. Utilisation d'un adhésif tel que défini dans l'une quelconque des revendications 1 à 3 dans des tapis, des revêtements de sol en linoléum, caoutchouc ou textile, des parquets ou des fenêtres vitrées isolantes.
